(19) Europäisches Patentamt
European Patent Office
Office européen des brevets

(11) **EP 2 101 170 A1**

(12) **EUROPEAN PATENT APPLICATION**

(43) Date of publication:
**16.09.2009 Bulletin 2009/38**

(51) Int Cl.:
*G01N 22/04* (2006.01)　　*G01N 33/34* (2006.01)

(21) Application number: **08152598.2**

(22) Date of filing: **11.03.2008**

(84) Designated Contracting States:
**AT BE BG CH CY CZ DE DK EE ES FI FR GB GR HR HU IE IS IT LI LT LU LV MC MT NL NO PL PT RO SE SI SK TR**
Designated Extension States:
**AL BA MK RS**

(71) Applicant: **Nederlandse Centrale Organisatie Voor Toegepast Natuurwetenschappelijk Onderzoek TNO 2628 VK Delft (NL)**

(72) Inventors:
• **Maas, Arnoldus Petrus Maria**
  **2586 TH Den Haag (NL)**
• **van de List, Jozef Fransiscus Maria**
  **2201 SM Noordwijk (NL)**

(74) Representative: **Hatzmann, Martin**
  **Vereenigde**
  **Johan de Wittlaan 7**
  **2517 JR Den Haag (NL)**

(54) **Method, system and computer program for contactless measuring a moisture percentage**

(57)　The invention relates to a method for contactless measuring a moisture percentage in a substantially flat structure (3) by transmitting an incident electromagnetic wave towards the substantially flat structure receiving an electromagnetic wave reflected by the structure determining a reflection coefficient from characteristics of the reflected electromagnetic wave and mapping the reflection coefficient to a moisture percentage.

Fig. 1

**Description**

The invention relates to a method for contactless measuring a moisture percentage in a substantially flat structure, comprising the step of transmitting an incident electromagnetic wave towards the substantially flat structure.

Such a contactless measuring method is e.g. known in the field of determining a moisture level in paper during its manufacturing process. By performing an electromagnetic transmission measurement, electromagnetic properties of the intermediate paper, between a transmitting and a receiving unit, can be derived, so that an actual moisture level in the paper can be estimated.

The drying process of the paper is a crucial aspect in the manufacturing process. Therefore, properly setting machine parameters, such as speed, heating and pressure, at different locations on the paper producing machine is important for obtaining a desired paper quality. In the process of choosing right parameters, paper moisture information is highly desired. Sensing devices for sensing the paper moisture content are hardly employed because known sensing devices do not work very well at the specific locations in the paper producing machine. As an example, it is generally not desired or possible to allocate measurement devices on both sides of the paper, thereby rendering the above-described contactless measuring method useless for industrial paper application.

Alternatively, optical measurement systems are known for estimating a paper moisture level in the paper. However, such optical systems are costly, not always reliable and expensive in maintenance. Also contact measurement systems are known which, however, are not suitable for performing measurements in high velocity paper producing machines.

It is an object of the invention to provide a measuring method according to the preamble wherein at least one of the disadvantages identified above, is counteracted. In particular, it is an object of the invention to provide a versatile method for contactless measuring a moisture percentage according to the preamble. Thereto, according to the invention, the method further comprises the steps of:

-    receiving an electromagnetic wave reflected by the substantially flat structure;
-    determining a flat structure reflection coefficient from characteristics of the reflected electromagnetic wave; and
-    mapping the reflection coefficient to a moisture percentage.

By performing a reflection measurement and by mapping the flat structure reflection coefficient to a moisture percentage, a method is obtained wherein measurement equipment is merely required on one side of the substantially flat structure to be characterized. As a consequence, a versatile method is obtained using a relatively compact measurement system, thereby enabling proper measurements at locations in a paper producing machine that are not or hardly available for measuring purposes when using prior art measuring devices.

Further, by employing an electromagnetic contactless measurement system, measurements can advantageously be performed in relatively low environmental temperature and relatively high environmental temperature, dry as well as in high humidity, harsh environment conditions. Moreover, during a measurement process a very low level of radiation is generated while it is able to potentially measure with resolutions up to a few cm. In addition, the equipment is potentially low cost, robust and requires less maintenance costs.

By allowing the use of moisture measurement systems at various locations in the paper producing apparatus, the production process can be monitored and controlled in an enhanced way, thereby enabling a more stable process leading to a better and more consistent quality of the final product. Further, manufacturing costs might be reduced.

In an elegant embodiment according to the invention, the step of determining the flat structure reflection coefficient comprises filtering the reflected electromagnetic wave from an ensemble of received signals originating from the original incident electromagnetic wave. By retrieving the reflected electromagnetic wave from the received signal, a relatively accurate reflection coefficient measurement can be performed, thereby improving the overall accuracy of the method. Alternatively, the reflection coefficient is determined from the unfiltered received signal, e.g. if the substantially flat structure is the only substantially reflecting object in the neighbourhood of the measurement equipment.

The invention further relates to a measurement system.

The invention also relates to a computer program product.

Other advantageous embodiments according to the invention are described in the following claims.

By way of example only, embodiments of the present invention will now be described with reference to the accompanying figures in which

Fig. 1 shows a schematic side view of a measurement system according to the invention;
Fig. 2 shows a diagram of incident and reflected signals;
Fig. 3 shows a diagram of a mixed signal;
Fig. 4 shows a spectral diagram of the mixed signal in Figure 3;
Fig. 5 shows a diagram of paper reflection coefficients as a function of a relative moisture content; and

Fig. 6 shows a block diagram of the measurement system shown in Figure 1.

The figures are merely schematic views of preferred embodiments according to the invention. In the figures, the same reference numbers refer to equal or corresponding parts.

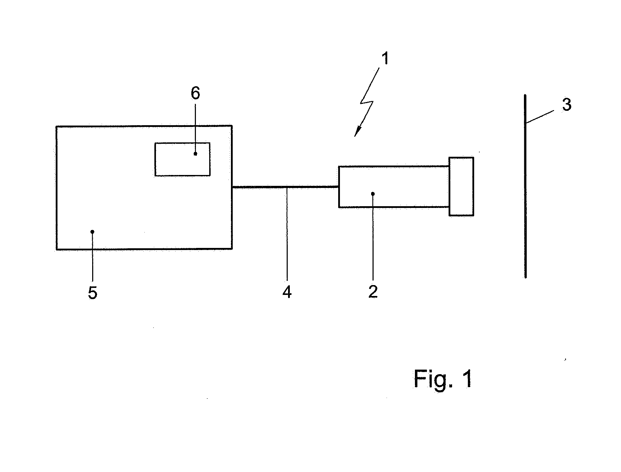

Figure 1 shows a schematic side view of a measurement system 1 according to the invention. The measurement system 1 is arranged for contactless measuring a moisture percentage in a paper 3. Thereto, the system 1 comprises an integrated transmitting unit and receiving unit for transmitting an incident electromagnetic wave towards a paper 3 and for receiving an electromagnetic wave reflected by the paper 3, respectively. The integrated transmitting unit and receiving unit including some sort of antenna for radiating the incident electromagnetic wave, is implemented as a broadband FMCW radar 2 having a low radiated power to meet local regulatory requirements. The radar 2 is, via a connection 4, connected to a computer system 5 comprising a processor 6 for determining a paper reflection coefficient from characteristics of the reflected electromagnetic wave received by the radar 2. Further, the processor 6 is arranged for mapping the paper reflection coefficient to a moisture percentage. As a result, a relative moisture content of the paper 3 can be measured.

By integrating the transmitting unit and the receiving unit with the antenna, costs are reduced, both regarding components as well as for measures that would have to be taken into account for processing the remote signals of the transmission unit and receiving unit. Further, a very compact measurement system can be realized allowing the use of multiple measurement systems at various locations in a paper producing machine. In a particular embodiment, however, the transmitting unit and the receiving unit have been implemented separately, e.g. if the measurement is composed of standard components in a simple hierarchy.

The radar 2 is facing the paper 3 that is oriented in a substantial vertical direction, so that the radar 2 is focussed in a substantially horizontal direction, substantially transversely with respect to the orientation of the paper 3. A typical distance between the radar 2 and the paper is e.g. circa 20 cm to 40 cm. By using radar techniques, in principle no moving parts are required for performing the measurements, thereby saving costs and increasing the reliability of the system.

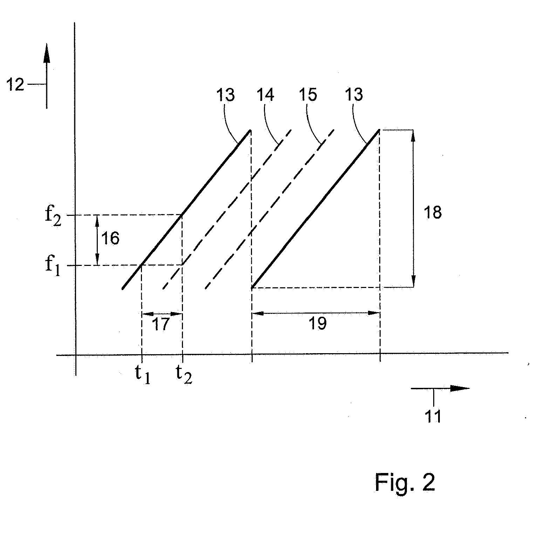

The radar 2 transmits a frequency modulated continuous wave (FMCW) signal, meaning that a central frequency of a continuous wave signal is varied according to a predetermined pattern. Figure 2 shows a diagram of incident and reflected signals. The diagram shows the frequency behaviour 12 of the incident signal 13 as a function of time 11. The frequency pattern shows a periodic linear saw tooth profile having an exemplary frequency sweep $F_{sweep}$ 18 of 2.5 GHz and an exemplary sweep time $T_{sweep}$ 19 of 1 ms. The diagram further shows the frequency behaviour 14, 15 of an ensemble of received signals originating from the original incident electromagnetic wave after reflection phenomena. In the exemplary diagram, the ensemble comprises a first reflected signal 14 being reflected by the paper 3 and a second reflected signal 15 being reflected by an object that is more remote from the radar 2 than the paper 3. Due to travel times, the first reflected signal 14 has a smaller time delay with respect to the original transmitted signal 13 than the time delay of the second reflected signal 15. By discriminating in time delays of the respective reflected signals with respect to the original signal, the first reflected signal 14 can be filtered from the ensemble of signals that are received by the radar 2 after transmission of the original signal 13.

It is noted that the frequency sweep $F_{sweep}$ can be set to another value, e.g. in a range between less than 1 GHz and substantially 10 GHz or even higher, wherein 10 GHz corresponds to a distance resolution of approximately 1.5 cm. The frequency sweep $F_{sweep}$ can be located anywhere in the electromagnetic spectrum between 1 GHz and 100 GHz, depending on local regulatory requirements. Further, the sweep time $T_{sweep}$ can be set to another value, e.g. in a range between substantially 10 $\mu$s and substantially 1 s, depending on measurement speed requirements and other system related requirements or limitations. Further, various antenna types can be used for transmitting and receiving the electromagnetic waves, e.g. ranging from an open waveguide radiating element or standard waveguide elements to more sophisticated wide frequency band antenna radiating elements based on any known antenna technology.

On the basis of the time delay 17 between the original signal transmission time t1 and the reflected signal receipt time t2, the distance between the paper and the radar can be estimated. Similarly, said distance is also related to an instant frequency difference 16, also called beatnote frequency $f_{beat}$, between a frequency $f_2$ of the original signal 13 and a frequency $f_1$ of the reflected signal 14 at a particular time instant $t_2$. Though the original signal is transmitted before receiving the reflected signal, the implementation of the measuring method thus admits that in practice both signals can be compared simultaneously for retrieving the distance information without using a memory for storing the signals. The beatnote frequency $f_{beat}$ is related to the distance information via the relation

$$f_{beat} = 2\ D\ F_{sweep}\ /\ (c\ T_{sweep})\ , \hspace{2cm} (1)$$

wherein D denotes the distance between the radar and the paper and c is the speed of light.

By increasing the FMCW sweep, $F_{sweep}$, the signal reflected by the paper can be distinguished from the ensemble of received signals more accurately, thereby improving the measurement.

It is noted that it is not necessary to explicitly compute the distance between the paper and the radar. However, the distance information can be used to filter the desired reflected signal from the ensemble of received signals.

It is further noted that the distance between the paper 3 and the radar 2 can be estimated otherwise, e.g. by performing an acoustic measurement. Further, said distance can be measured by hand and be fed into the measurement system, e.g. if the measurement system is stationary and the distance between the paper and the radar remains constant over time.

In principle, the transmitted signal can be modulated otherwise, e.g. as an amplitude modulated continuous wave wherein the amplitude modulations has a periodic pattern, so that the amplitude forms a basis for discriminating a desired reflected signal from the ensemble of signals that is received after transmission of the original signal.

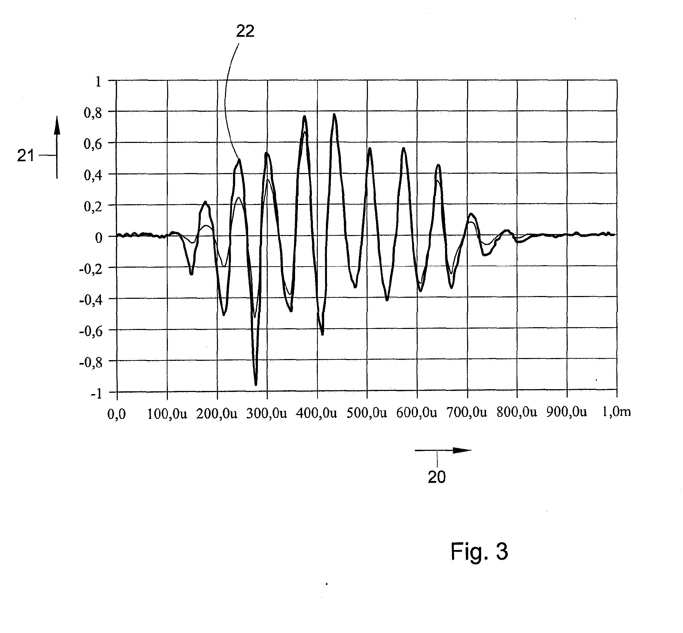

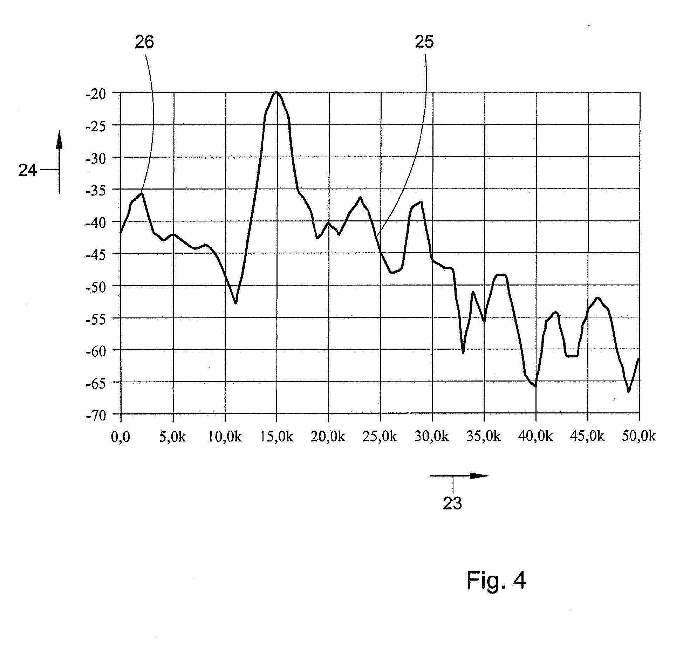

In order to process the reflected signal data, the original signal and the signal reflected by the paper are fed to a mixer in order to generate the beatnote frequency signal, which is then subjected to a Fast Fourier Transform (FFT). Figures 3 and 4 show a diagram of a mixed signal in the temporal domain and in the spectral domain, respectively. In particular, Figure 3 shows the amplitude 21 of the time dependent mixed signal 22 as a function of the time 20, while Figure 4 shows the amplitude spectrum 24 of the transformed mixed signal 25 as a function of the frequency 23. The spectral mixed signal comprises a number of peaks corresponding with reflected signals in the ensemble of received signals and one or more low frequency peaks 26 that reflect measurement system characteristics. The spectral information of the reflected signal of interest being reflected by the paper, can be retrieved by considering the amplitude of the peak near a frequency $f_{beat}$ corresponding with the distance D between the radar and the paper using relation (1). By filtering the desired reflected signal, other signals can advantageously be suppressed.

By determining the amplitude of the frequency $f_{beat}$ related to the amplitude of the original signal, a reflection coefficient can be determined that is indicative of the relative moisture content in the paper. As a next step, the paper reflection coefficient is than mapped to a moisture percentage by selecting a moisture percentage value corresponding with the determined reflection coefficient in accordance with a predetermined relationship between the moisture percentage value and the paper reflection coefficient. Such a predetermined relationship can e.g. be stored in a memory after a series of measurements that have been performed wherein the moisture percentage value in the paper is measured directly, e.g. by measuring its weight.

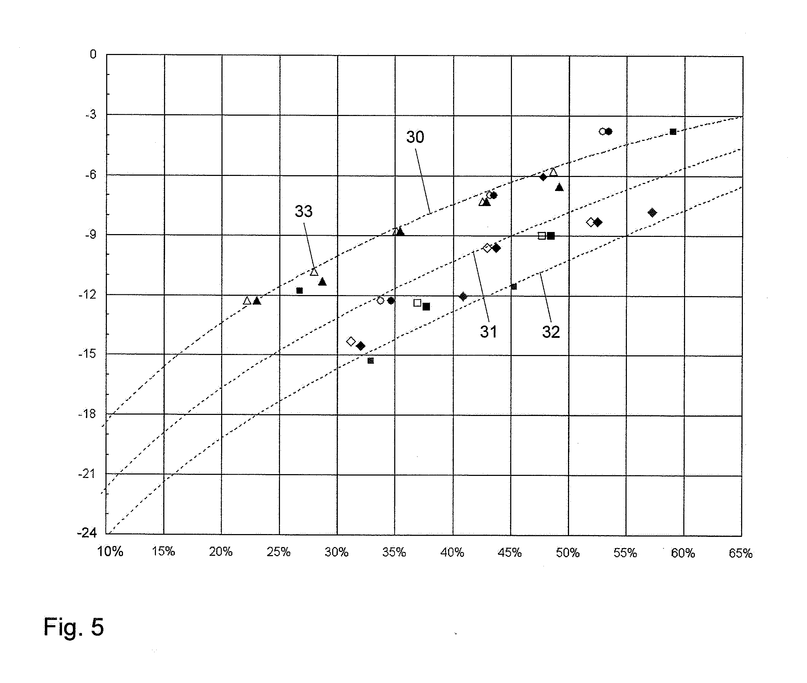

Figure 5 shows a diagram comprising a multiple number of curves 30, 31, 32 representing a relationship between a moisture percentage 34 and the paper reflection coefficient 35 [dB]. Said relationship depends e.g. on paper material and paper thickness. In particular, moisture percentage value measurements 33 have been performed and theoretical and interpolation curves have been drafted. By selecting a specific curve associated with a particular paper material and thickness, a paper reflection coefficient measurement can be mapped to an actual moisture percentage in the paper.

The measurement system according to the invention is well suited for performing moisture level measurements as the relative permittivity $\varepsilon_r$ of water is in the order of 60 while the relative permittivity $\varepsilon_r$ of paper is in the order of 2 or 3, so that the presence of water in the paper significantly modifies the electromagnetic reflection coefficient. If the paper is dry, the reflection coefficient is relatively low while wet paper provides a relatively high reflection coefficient.

In order to perform a paper reflection coefficient measurement, the method according to the invention optionally comprises a calibration step by using a metal plate as the substantially flat structure, so that an actual measurement can be related with measurement data retrieved in the calibration step.

In an advantageous manner, the calibration coefficient and an actual reflection coefficient can be interrelated to a spectral characteristic that is inherent to devices used for performing the measurement method. In particular, the calibration coefficient and the actual reflection coefficient can be interrelated to one or more of the above-mentioned low frequency peaks 26 that reflect measurement system characteristics for computing the paper reflection coefficient. As a consequence, the drifting effects of the measurement system can at least partially be compensated, thereby allowing a relatively long calibration interval. In an alternative embodiment, the calibration coefficient is stored for directly comparing with an actual reflection coefficient.

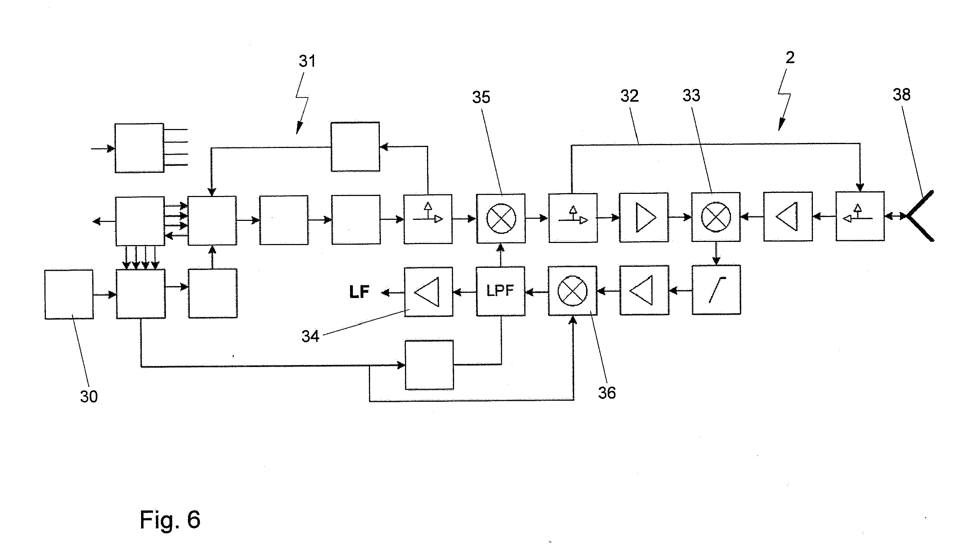

Figure 6 shows a block diagram of the radar 2 shown in Figure 1. The system comprises a local reference oscillator 30 for generating the FMCW signal employing a phase lock loop circuit 31. Said FMCW signal is fed via a connection line 32 towards the antenna element 38. Further, the same antenna element 38 is used for receiving the ensemble of reflected signals. Both, the original signal and the reflected signals are mixed in a mixer 33. A low pass filter 34 filters the mixed signal for further processing, such as conversion to a digital signal and applying an FFT routine. The system further comprises additional mixers 35, 36 for performing a BSPK modulation and demodulation procedure.

Advantageously, a BSPK modulation and demodulation technique is used to counteract low-frequency mixer noise and reduce any influence of the reflection coefficient of the radar antenna element 38 from limiting the receiver sensitivity. By periodically applying a 180° phase shift in the transmitted signal, an overlap in the transmitted and reflected signal is a measure for the distance between the radar and the paper. A signal that is proportional with said overlap can advantageously be used to compensate for losses due to the spatial extend of the electromagnetic waves in the intermediate medium, in the described embodiment air, between the radar and the paper. The amount of losses depend on the distance between the radar and the paper. Hence, a receiver-gain is proportional to a delay in the reflected signal, making the receiver output signal amplitude substantially independently of the distance D between the radar and the paper.

Experiments show that the measurement system according to the invention provides most accurate measurement signals when the material composition of the substantially flat structure is substantially homogenous.

It is noted that the step of determining a flat structure reflection coefficient from an electromagnetic wave that has been reflected by a substantially flat structure after being transmitted towards the structure, e.g. from the amplitude information of the beatnote frequency, and the step of mapping the reflection coefficient to a moisture percentage, can be performed either by dedicated hardware or by standard hardware that is loaded with software suitable for performing the data processing tasks, i.e. a computer system 5 and a processor 6 in the measurement system 1.

The invention is not restricted to the embodiments described herein. It will be understood that many variants are possible.

The Fast Fourier Transform can be replaced by another Transform for determining the reflection coefficient in another numerical domain. Further, instead of applying a spectral analysis after performing a mixing operation of the original signal and the reflected signal, also other techniques can in principle be applied for evaluating the reflection coefficient, e.g. by analyzing the relevant signals in the time domain. Analogue pre-processing of the beatnote frequency signal, e.g. extracting the paper reflection coefficient from the spectrum by using band-pass filtering, are also envisaged.

When a multiple number of measurement systems are applied in a single measurement environment, one or more processors connected to the transmitting and receiving units can be integrated, instead of using separate processors in combination with each measurement system. In a particular situation, a single processor can be used to process the signals of a multiple set of measurement systems.

In addition, the measurement method according to the invention is not restricted to measuring moisture levels in paper, such as water or dirt moisture. Also other moisture percentages in other substantially flat structures can be measured, such as a relative moisture level in cardboard. Also, the method is not restricted to measuring a water percentage, but can also be applied to measurements of other liquids in a substantially flat structure. Further, pressing processes can be monitored or the moisture level in tobacco layers can be measured. Further various other substantially flat structures can be screened, such as clothing materials during a painting, washing and/or drying process.

Further such variants will be obvious for the man skilled in the art and are considered to lie within the scope of the invention as formulated in the following claims.

## Claims

1. Method for contactless measuring a moisture percentage in a substantially flat structure, comprising the steps of:

    - transmitting an incident electromagnetic wave towards the substantially flat structure;
    - receiving an electromagnetic wave reflected by the substantially flat structure;
    - determining a flat structure reflection coefficient from characteristics of the reflected electromagnetic wave; and
    - mapping the reflection coefficient to a moisture percentage.

2. Method according to claim 1, wherein the determining step comprises filtering the reflected electromagnetic wave from an ensemble of received signals originating from the original incident electromagnetic wave.

3. Method according to claim 2, wherein the filtering step comprises estimating a distance between the substantially flat structure and a transmitting device transmitting the incident wave.

4. Method according to any of the previous claims, wherein the incident electromagnetic wave is a frequency modulated continuous wave.

5. Method according to any of the previous claims, wherein the distance between the substantially flat structure and the transmitting device is estimated by determining a frequency difference between the incident wave and the reflected wave.

6. Method according to any of the previous claims, wherein the determining step further comprises the steps of:

    - mixing the incident wave and the reflected wave to obtain a mixed signal; and

- performing a spectral analysis on the mixed signal.

7. Method according to any of the previous claims, further comprising a calibration step by using a metal plate as the substantially flat structure.

8. Method according to any of the previous claims, wherein the calibration reflection coefficient and an actual reflection coefficient are interrelated to a spectral characteristic that is inherent to devices used for performing the measurement method.

9. Method according to any of the previous claims, wherein the mapping step is performed by selecting a moisture percentage value corresponding with the determined reflection coefficient in accordance with a predetermined relationship between the moisture percentage value and the reflection coefficient.

10. Measurement system for contactless measuring a moisture percentage in a substantially flat structure, comprising:

- a transmitting unit for transmitting an incident electromagnetic wave towards the substantially flat structure;
- a receiving unit for receiving an electromagnetic wave reflected by the substantially flat structure;
- a processor for determining a flat structure reflection coefficient from characteristics of the reflected electromagnetic wave and for mapping the flat structure reflection coefficient to a moisture percentage.

11. Measurement system according to claim 10, wherein the transmitting unit and the receiving unit are focussed in a direction substantially transversely with respect to the orientation of the substantially flat structure.

12. Measurement system according to claim 10 or 11, wherein the transmitting unit and the receiving unit have been integrated.

13. Computer program product, comprising computer readable code for causing a processor to perform a method of contactless measuring a moisture percentage in a substantially flat structure, comprising the steps of:

- determining a flat structure reflection coefficient from characteristics of a received electromagnetic wave that has been reflected by a substantially flat structure after being transmitted towards the structure; and
- mapping the flat structure reflection coefficient to a moisture percentage.

Fig. 1

Fig. 2

Fig. 3

Fig. 4

Fig. 5

Fig. 6

European Patent
Office

**EUROPEAN SEARCH REPORT**

Application Number

EP 08 15 2598

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 01/02841 A (NELES PAPER AUTOMATION OY [FI]; JAKKULA PEKKA [FI]; DAHLSTROEM ILKKA [ ]) 11 January 2001 (2001-01-11) | 1,2,4, 6-12 | INV. G01N22/04 G01N33/34 |
| Y | * page 3, line 29 - page 5, line 24; figure 1 * | 3,5,13 | |
| Y | US 5 315 258 A (JAKKULA PEKKA [FI] ET AL) 24 May 1994 (1994-05-24) * column 2, lines 21-34; figure 1 * | 3 | |
| Y | * column 5, lines 44-59 * | 5 | |
| Y | * column 6, lines 28-30 * | 13 | |
| X | US 5 256 978 A (ROSE MITCHELL [US]) 26 October 1993 (1993-10-26) * column 1, lines 21,22 * * column 4, lines 3-41; figure 1 * | 1,7-12 | |
| A | GB 883 828 A (BELOIT IRON WORKS) 6 December 1961 (1961-12-06) * page 2, line 91 - page 3, line 78; figure * | 1-13 | |

TECHNICAL FIELDS SEARCHED (IPC)

G01N

The present search report has been drawn up for all claims

| Place of search | Date of completion of the search | Examiner |
|---|---|---|
| The Hague | 20 August 2008 | Wilhelm, Jörg |

EPO FORM 1503 03.82 (P04C01)

## ANNEX TO THE EUROPEAN SEARCH REPORT
## ON EUROPEAN PATENT APPLICATION NO.

EP 08 15 2598

This annex lists the patent family members relating to the patent documents cited in the above-mentioned European search report.
The members are as contained in the European Patent Office EDP file on
The European Patent Office is in no way liable for these particulars which are merely given for the purpose of information.

20-08-2008

| Patent document cited in search report | | Publication date | Patent family member(s) | | Publication date |
|---|---|---|---|---|---|
| WO 0102841 | A | 11-01-2001 | AU<br>FI | 5832400 A<br>991548 A | 22-01-2001<br>05-04-2001 |
| US 5315258 | A | 24-05-1994 | NONE | | |
| US 5256978 | A | 26-10-1993 | NONE | | |
| GB 883828 | A | 06-12-1961 | NONE | | |

EPO FORM P0459

For more details about this annex : see Official Journal of the European Patent Office, No. 12/82